# EUROPEAN PATENT SPECIFICATION

(11) **EP 2 754 444 B1**
(45) Date of publication and mention of the grant of the patent: **19.06.2019**
(21) Application number: 12830605.7
(22) Date of filing: 08.08.2012
(51) Int. Cl.: A23K 20/163, A23L 29/30, A23L 33/10, A23L 33/125, A23L 2/52, A61K 31/7004, C07H 3/02

(54) **COMPOSITION FOR IMPROVING IN VIVO METABOLISM PARAMETER**
ZUSAMMENSETZUNG ZUR VERBESSERUNG VON IN-VIVO-STOFFWECHSELPARAMETERN
COMPOSITION POUR AMÉLIORER UN PARAMÈTRE DU MÉTABOLISME IN VIVO

(30) Priority: 06.09.2011 JP 2011194037
(43) Date of publication of application: 16.07.2014
(73) Proprietor: Rare Sugar Foods, Llc., Marugame-shi, Kagawa 763-0042 (JP); Matsutani Chemical Industry Co., Ltd., Itami-shi, Hyogo 6648508 (JP)
(72) Inventor: YAMADA, Koji, Itami-shi Hyogo 664-8508 (JP); HAYASHI, Noriko, Itami-shi Hyogo 664-8508 (JP); YAMADA, Takako, Itami-shi Hyogo 664-8508 (JP)
(74) Representative: TBK
(86) International application number: PCT/JP2012/070226
(87) International publication number: WO 2013/035479

(56) References cited:
- JP-A- 2010 018 528
- NORIKO HAYASHI ET AL: "Study on the Postprandial Blood Glucose Suppression Effect of D-Psicose in Borderline Diabetes and the Safety of Long-term Ingestion by Normal Human Subjects", BIOSCI. BIOTECHNOL. BIOCHEM., vol. 74, no. 3, 7 March 2010 (2010-03-07), pages 510-519, XP002739230,
- HOSSAIN,M.A. ET AL.: 'Rare sugar D-psicose improves insulin sensitivity and glucose tolerance in type 2 diabetes Otsuka Long-Evans Tokushima Fatty (OLETF) rats' BIOCHEMICAL AND BIOPHYSICAL RESEARCH COMMUNICATIONS vol. 405, no. 1, February 2011, pages 7 - 12, XP028138620
- MATSUO,T. ET AL.: 'Dietary D-psicose, a C-3 epimer of D-fructose, suppresses the activity of hepatic lipogenic enzymes in rats' ASIA PACIFIC JOURNAL OF CLINICAL NUTRITION vol. 10, no. 3, 2001, pages 233 - 237, XP002430681
- MATSUO,T. ET AL.: 'Less body fat accumulation with D-psicose diet versus D-fructose diet' JOURNAL OF CLINICAL BIOCHEMISTRY AND NUTRITION vol. 30, 2001, pages 55 - 65, XP003003027
- MASANORI FUKUSHIMA THE MERCK MANUAL 2007, pages 201 - 202, XP008173258
- MATSUO,T. ET AL.: 'Ninety-day oral toxicity study of rare sugar syrup in male Wistar rats' CURRENT TOPICS IN TOXICOLOGY vol. 7, 2011, pages 41 - 49, XP008172808
- MATSUO,T. ET AL.: 'The 90-day oral toxicity of D-psicose in male Wistar rats' JOURNAL OF CLINICAL BIOCHEMISTRY AND NUTRITION vol. 50, no. 2, March 2012, pages 158 - 161, XP055146058
- , Retrieved from the Internet: URL:https://www.mayoclinic.org/diseases-co nditions/diabetes/diagnosis-treatment/drc- 20371451?p=1 [retrieved on 2018-05-09]
- "Definition and Diagnosis of Diabetes Mellitus and Intermediate Hyperglycaemia, WHO Report 2006", , 14 January 2006 (2006-01-14), Retrieved from the Internet: URL:https://www.who.int/diabetes/publicati ons/diagnosis_diabetes2006/en/

## Description

### Technical Field

The present invention relates to a composition that contains D-psicose as an active ingredient for improvement of biometabolism parameters.

As used herein, the term
"uric acid level" will be
referred to as "biometabolism parameter".

### Background Art

A uric acid level represents an important biometabolism parameter. A blood uric acid concentration above 7 mg/dL indicates hyperuricemia.

D-psicose has low calories (Patent Document 1, and Non-Patent Documents 1 and 2), and is hardly converted into energy. In addition, 70% of the D-psicose ingested is directly absorbed but excreted into urine. The remaining 30% reportedly reaches the large intestine, and excreted into feces essentially without being utilized by enteric bacteria. Functionally, D-psicose has been reported to have postprandial glucose level suppressing effect (Non-Patent Document 2) . The mechanisms by which D-psicose exhibits such effect are mainly through inhibition of sugar breakdown, and promotion of glucose uptake in the liver. Other reported characteristics of D-psicose include fat reduction (Patent Document 2). However, the mechanism of action remains elusive.

Alkali isomerization is the industrial sugar isomerization method described in Patent Document 3.

NORIKO HAYASHI et al., "Study on the Postprandial Blood Glucose Suppression Effect of D-Psicose in Borderline Diabetes and the Safety of Long-Term Ingestion by Normal Human Subjects", BIOSCI. BIOTECHNOL. BIOCHEM., (20100307), vol. 74, no. 3, pages 510 - 519, reports on a clinical study, which was conducted to investigate the safety and effect of D-psicose on postprandial blood glucose levels in adult men and women, including borderline diabetes patients.

### Citation List

### Patent Documents

Patent Document 1: JP-A-2007-51137
Patent Document 2: JP-A-2010-18528
Patent Document 3: WO2010/113785
Patent Document 4: JP-A-6-125776

### Non-Patent Documents

Non-Patent Document 1: Metabolism. 59(2), 206-214, 2010
Non-Patent Document 2: J Nutr Sci Vitaminol. 48:77-80.
Non-Patent Document 3: J. Am. Chem. Soc. 1955. 77. 3323-3325
Non-Patent Document 4: Biochemistry. 16 (10):2169-75, 1977

### Summary of the Invention

### Problems that the Invention is to Solve

There is no report that describes the effect of D-psicose on the uric acid level.

There is a strong need for the development of a drug that can effectively lower the uric acid level, and a health beverage and food, or an animal feed that can lower the uric acid level, through daily ingestion. An object of the present invention, then, is to provide a composition for use in lowering the uric acid level,
a beverage and food, a feed, and a medicinal product for use in lowering the uric acid level.

### Means for Solving the Problems

The present inventors conducted intensive studies to achieve the foregoing objects, and found that D-psicose has the effect to lower the biometabolism parameter uric acid level.

The present invention was completed after determining the effective dose and the effective period of such D-psicose.

Specifically, the present invention provides a use as defined in claims 1 and 2.

### Advantage of the Invention

The present invention determined the effective dose and the effective period for lowering the uric acid level.

The effective dose and the effective period were found to be continuous administration of 5 g or more per intake, in a daily dose of 15 g/day for 2 weeks or longer than 2 weeks, as demonstrated in Examples.

The present invention also can provide a use that can effectively lower the uric acid level,
and a health beverage and food or an animal feed that can lower the uric acid level. Specifically, the present invention can provide a use for lowering the uric acid level, a beverage and food, a feed, and a medicinal product for use in lowering the uric acid level.

### Brief Description of the Drawing

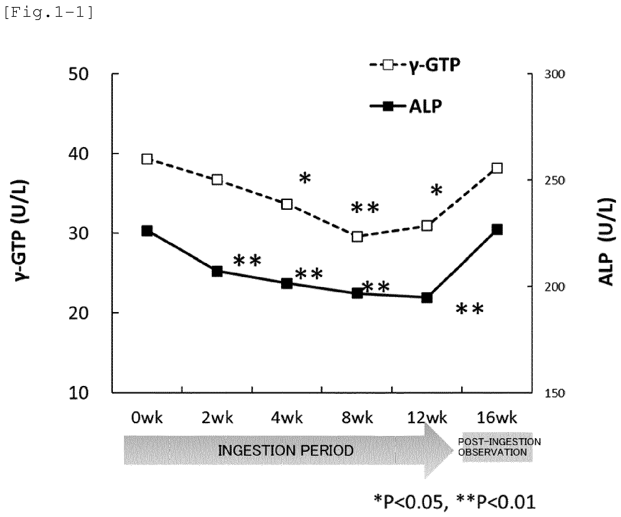

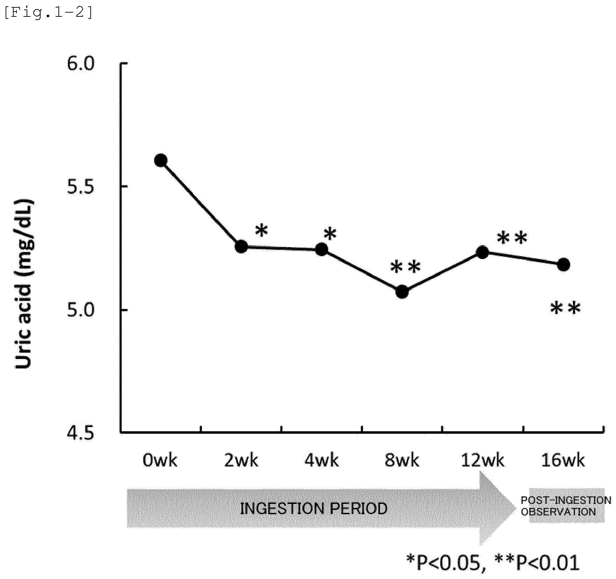

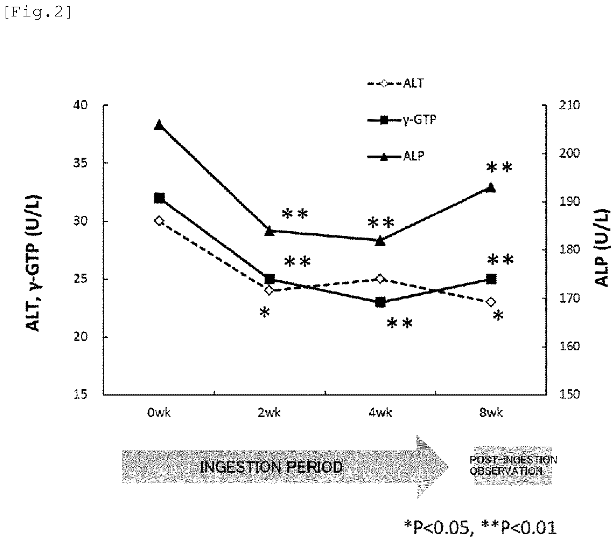

FIG. 1-2 is a diagram representing changes in the uric acid level of Example 1.

The specific function of the D-psicose-containing material of the present invention is the uric acid level lowering effect.

The D-psicose of the present invention may be one that is enzymatically or chemically produced from D-fructose, or indirectly from D-glucose through epimerization, or may be one that is extracted from plants, and may be purified completely, or may contain trace amounts of impurities present after the production. For example, the D-psicose of the present invention can be prepared relatively easily by using the technique that uses epimerase (see, for example, Patent Document 4). The D-psicose of the present invention also may be chemically produced (Non-Patent Document 3).

The D-psicose solution obtained may be purified using a method such as deproteination, decolorization, and desalting, as required, and may be concentrated to be obtained as a syrup-like D-psicose product. The product may be fractionated and purified by column chromatography, upon which a standard preparation with a purity of 99% or more can easily be obtained. The product D-psicose can directly be used as a monosaccharide.

The D-psicose also may be used as a mixed sugar, for example, a mixed sugar containing D-glucose, D-fructose, or other rare sugars (e.g., allose).

The D-psicose may be used as a D-psicose and/or a derivative thereof.

The D-psicose derivatives used in the present invention are described below. A derivative is a compound obtained after the conversion of a starting compound through a chemical reaction that changes the molecular structure. There is a wide variety of D-psicose derivatives along with derivatives of other hexoses. Non-limiting examples include amino sugars (sugars substituted with a NH₂ group at an OH group of the sugar molecule; glucosamine, chondrosamine, and glycoside). In the case of monosaccharide physiological effects, the main mechanism of action depends on the specificity to enzyme, and the sugar conformation is particularly important. For example, fructose is the main substrate of the phosphorylation by the fructokinase enzyme in liver. This enzyme also phosphorylates D-tagatose and D-psicose, C-3 isomers of fructose, but their phosphorylation rates depend on the conformation (Non-Patent Document 4). The reaction rate of fructokinase is affected particularly by the trans/axial relationship of the 2-, 3-, 4-, and 5-position carbon atoms. It can thus be speculated that the effect of the present invention can be sufficiently obtained even with the derivatives, provided that the conformation is maintained to some extent.

The effective dose of D-psicose is 5 g or more per intake. In Example 1, the dosage is 5 g per intake, three times each day.

The ingestion period of D-psicose is 2 weeks, or longer than 2 weeks.

The D-psicose used in the present invention may have any form, including a solid form such as a powder, a fine powder, a granule, a crystal, and a tablet, and a form of an aqueous solution or a solution. The method of production is not particularly limited either. It is also possible to contain non-sweet or non-taste components such as a bulking agent and a carrier, provided that such addition does not interfere with the purposes of the present invention.

The present invention also provides the use of a beverage and food obtained using the D-psicose-containing material of the present invention, particularly materials with functions.

The beverage and food of the present invention encompasses a range of foods that require sweetness in general, including drinks, candies, frozen desserts, yogurts, and chocolates. Other examples of products with the imparted sweetness include medicinal products and oral compositions.

For example, the present invention can be advantageously used as a sweetener for various flavor enhancers such as soy sauce, a soy sauce powder, *miso,* a *miso* powder, *moromi, hishio, furikake,* mayonnaise, dressing, vinegar, *sanbaizu* (vinegar mixture), a sushi vinegar powder, a taste enhancer for Chinese food, *tentsuyu* (dipping sauce for tempura), *mentsuyu* (dipping sauce for noodles), sauce, ketchup, sauce for grilled meat, curry block, a stew powder, a soup powder, a soup stock powder, a combination flavor enhancer, *mirin* (sweet sake seasoning made from rice), *shin-mirin* (sweet sake seasoning made from coarse cereals), table sugar, and coffee sugar. Other possible advantageous uses include taste improvers, and quality improvers.

The present invention also can be used as an additive for various foods and beverages, including Japanese confectioneries such as *senbei, arare, okoshi,* rice cake, *manju, uiro,* red bean pastes, *yokan, mizuyokan, kingyoku,* jelly, castella, and candies; Western confectioneries such as bread, biscuits, crackers, cookies, pies, pudding, butter cream, custard cream, profiterole, waffles, sponge cake, doughnuts, chocolates, chewing gums, caramels, and candies; frozen desserts such as icecream and sherbet; syrups such as syruped fruits and *korimitsu* (syrup for shaved ice); pastes such as flower pastes, peanut pastes, and fruit pastes; processed fruit and vegetable products such as jam and marmalade, syrup preserves, and sugar preserves; processed cereal products such as bread products, noodles, cooked rice products, and artificial meat; pickled vegetables such as *fukujinzuke, bettarazuke, senmaizuke,* and *rakkyozuke;* pickles powder products such as a *takuanzuke* powder and a *hakusaizuke* powder; animal products such as ham and sausage; fish products such as fish ham, fish sausage, *kamaboko, chikuwa,* and *tempura;* delicacies such as sea urchin, squid *shiokara, sukonbu, sakisurume,* and dried pufferfish with *mirin; tsukudani* products made from dried laver seaweed, *sansai,* dried shredded squid, small fish, and shellfish; side dishes such as *nimame,* potato salad, and a *konbu* roll; dairy product; bottled and canned products of fish meat, meat, fruits, and vegetables; alcohol products such as *gouseishu* (sake with additives), fruit wine, Western liquors, and liqueur; soft drinks such as coffee, hot chocolate, juice, carbonated drinks, lactic drinks, and lactobacillus beverages; premix powders such as a pudding mix, and a hotcake mix; and instant beverages and foods such as instant juice, instant coffee, instant red bean soup, and instant soup.

Specific examples of functional drinks include carbonated drinks such as coke, sports drinks, fruit juice, milk drinks, and tea drinks. Carbonated drinks, which can cause obesity when consumed in large quantities, represent a particularly desirable target.

The foods and beverages may be used as functional foods, dietary-supplements, or foods with health claims. The form of food is not particularly limited. For example, such food may be produced by using ordinary method, using materials such as proteins (e.g., milk proteins, soybean proteins, and egg albumin of high nutritional quality with a good balance of amino acids), degraded products of these proteins, egg white oligopeptides, soybean hydrolysate, and mixtures of amino acids alone. The foods and beverages may also be used, for example, in the form of a soft capsule, and a tablet.

Examples of the dietary-supplements or functional foods include processed products containing materials such as sugar, fat, trace element, vitamins, chemical agents, and flavors. Examples of such products include liquid food, defined formula diet, elemental diet, energy drink, capsule formulation, and enteral nutrient. These products, including food and beverages such as sports drinks, and nutritional supplement drinks may further contain nutritional additives and compositions, such as amino acids, vitamins, and minerals, and additives such as spice, flavor, and dye to enhance the nutritional balance and flavor.

Examples of products include common foods, foods for health use, clinical nutrition products, food materials, food materials for health use, clinical nutrition food materials, food additives, food additives for health use, clinical nutrition food additives, drinks, drinks for health use, clinical nutrition drinks, drinking water, drinking water for health use, clinical drinking water, drugs, pharmaceutical raw materials, feeds, and feeds for livestock and/or wild animals under treatment.

For food applications, the composition of the present invention may be used directly in a composition form, or in the form of a preparation, which may be of a form diluted in oil or the like, an emulsion form, or a form containing a carrier commonly used in food industry. The drinks may be non-alcohol drinks or alcohol drinks. Examples of the non-alcohol drinks include carbonated drinks, noncarbonated drinks such as fruit juice and nectar drink, soft drinks, sports drinks, tea, coffee, and hot chocolate. Examples of the alcohol drinks include common alcohol products, such as medicated liquor, chuhai, *umeshu,* beer, low-malt beer, and malt-free, beer-like alcoholic beverages.

For use as a food material or a food additive intended to improve the biological functions, the composition of the present invention may be used in the form of a solid agent such as a tablet, a capsule formulation, or a powder and a granule dissolved in a drink or the like, or in the form of a semi-solid such as a jelly, a liquid such as drinking water, or a high-concentration solution that is diluted before use.

The composition of the present invention may be appropriately added to food to provide a healthy diet or an invalid diet intended to improve biological functions. These may be appropriately mixed with additional components, such as vitamins, carbohydrates, dyes, and flavors commonly added to food. The food products may be ingested in any form, including a liquid form, and a solid form. Further, the composition of the present invention may be ingested in the form of a soft capsule formulation prepared by encapsulating the composition with gelatin or the like. Such capsules may be made of a gelatin coating prepared, for example, by dissolving the raw material gelatin by addition of water, and adding a plasticizer (such as glycerine, and D-sorbitol) to the gelatin solution.

The composition of the present invention is applicable to feeds for domestic animals, poultry, and pets. For example, the composition of the present invention may be mixed with dry dog foods, dry cat foods, wet dog foods, wet cat foods, semi-moist dog foods, poultry feeds, and feeds for domestic animals such as cows and pigs. Such feed itself may be prepared according to an ordinary method.

The therapeutic agent and the preventive agent may be used for non-human animals, including domestic mammals such as cows, horses, pigs, and sheep, poultry such as chicken, Japanese quail, and ostrich, pets such as reptiles, birds, and small mammals, and cultured fish.

The drug that takes advantage of the effects of the composition of the present invention may be used either alone, or may be mixed with a suitable additive such as a common excipient, a stabilizer, a preservative, a binder, and a disintegrant, and prepared into a suitable dosage form such as a liquid, a granule, a subtle granule, a powder, a tablet, a capsule formulation, a ball, an ointment, an adhesive skin patch, an epipastic, a spray, and an injection. These may be administered orally, transnasally, percutaneously, or intravenously.

A medicinal organic or inorganic solid, semi-solid, or liquid carrier, a solubilizer, or a diluent suited for oral administration, transnasal administration, transdermal administration, or intravenous administration may be used to prepare the composition of the present invention as a drug. Examples of the carriers usable for the drug containing the composition of the present invention include water, gelatin, lactose, starch, magnesium stearate, talc, animal and vegetable oils, benzyl alcohol, gum, polyalkylene glycol, petroleum resin, coconut oil, lanolin, and all other carriers used for medicinal applications. It is also possible to appropriately use a stabilizer, a wetting agent, and an emulsifier, and other adjuvant agents such as salts added to change osmotic pressure, or maintain the appropriate pH of a compounding agent.

Use of a soluble film has become common in the preparation of products such as cosmetics. For example, an edible soluble film has been used as a flavored film for purposes such as refreshing, and bad breath prevention. Examples of other possible applications that have been proposed include a moisturized cosmetic film produced as a mask, or as an emulsion by being dissolved in water. Potential use as a plaster containing an anti-inflammatory agent or the like also has been investigated. JP-A-2007-91696 proposes a soluble film that has excellent solubility and film characteristics, preferred for use as a wrapping material for food and products such as medicinal products, or as a food or medicinal product carrier for keeping the active ingredients. The composition of the present invention thus has use as a medicinal product or a quasi drug.

The present invention is described below in greater detail using Examples.

Clinical trials were performed with ethics committee approval to examine the effects of D-psicose as a composition for improving biometabolism parameters.

### Example 1

### <Experiment Methods>

Increasing incidence of lifestyle-related diseases, diabetes in particular, has been a problem in Japan. Clinical trials were conducted with recruited diabetic subjects, and biometabolism parameter changes due to D-psicose were investigated. The recruited subjects consisted of diabetes borderline subjects (12 subjects with fasting glucose levels of 110 to 126 mg/dl), and diabetes patients (6 type 2 diabetes patients undergoing drug treatment)(all subjects, males and females, were at least 20 years of age at the time of informed consent). Five grams of D-psicose was ingested per meal for 12 weeks. The D-psicose was purchased from Izumoring Co., Ltd. The subjects were tested after 0, 2, 4, 8, and 12 weeks from the start of the ingestion, and after 4 weeks from the end of the ingestion period (week 16, post-ingestion observation). The subjects were fasted after the dinner served on the day before the testing date. The subjects were measured for a uric acid level using the service of BML Inc. A paired t-test was used for statistical comparison before and after the ingestion. In the figure, significant difference is given by *: 0.05 < P or **: 0.01 < P.

### <Results>

The results are shown in FIG. 1-2 (uric acid level).

These results demonstrated that the repeated ingestion of D-psicose (about 5 g/intake) over the time period of about 2 to 4 weeks can improve the biological function parameter in humans. A significant decrease was observed in the uric acid level after two weeks from the ingestion. From the result of stratified analyses that the diabetes borderline patients showed a significant decrease after 2 weeks from the ingestion, it can be inferred that greater effects may be obtained particularly in pre-diabetic individuals.

### Example 3

### <Experiment Methods>

Fourteen Wistar male rats (CLEA Japan), 3 weeks of age, were used for experiments in groups of seven. The animals were fed on solid feed CE-2 (CLEA Japan) for 1 week, and grouped according to body weight after this acclimation period. The basic feed compositions are presented in Table 1. The rare sugar-containing syrup was prepared by feeding a 10% (w/v) isomerized sugar solution (high fructose corn syrop) a strongly basic ion-exchange resin at a temperature of 60°C (resin: Amberlite IRA900J [Cl]). The sugar composition of the reaction solution was 40% D-glucose, 31% D-fructose, and 7% D-psicose. The remaining 22% were other carbohydrates, including D-mannose, and D-allose.

The rats were maintained for 8 weeks with free access to feed and drinking water. The animals were then anesthetized with ether, and the abdominal cavity was opened in turn. Blood was collected from the subrenal aorta in the abdomen with a heparin-treated syringe. For blood plasma measurement the uric acid level was measured with commercially available kits. Experiment data are presented as mean value ± standard error. The mean value significant difference between the groups was calculated by using unpaired t-test.

**[Table 1]**

| | Isomerized sugar | Rare sugar-containing syrup |
|---|---|---|
| Casein | 175.5 | 175.5 |
| Cornstarch | 285.2 | 285.2 |
| Isomerized sugar¹ | 407.5 | 135.9 |
| Rare sugar-containing syrup² | 0 | 271.7 |
| Cellulose | 43.9 | 43.9 |
| Mineral mixture (AIN-76) | 30.7 | 30.7 |
| Vitamin mixture (AIN-76) | 8.8 | 8.8 |
| DL-methionine | 2.6 | 2.6 |
| Choline chloride | 1.8 | 1.8 |
| Powdered oil | 43.9 | 43.9 |
| Butylhydroxytoluene | 0.1 | 0.1 |
| | 1000 | 1000 |

### <Results>

As shown in Table 2, a significant decrease was observed in the uric acid level after the ingestion of the rare sugar-containing syrup.

This result demonstrated that the D-psicose-containing composition prepared by reisomerization of isomerized sugar with alkali had the tendency to improve the uric acid level.

**[Table 2]**

| | | Isomerized sugar | Rare sugar-containing syrup |
|---|---|---|---|
| | | | |
| Uric acid level | (mg/dL) | 20.7 ± 0.4 | 18.5 ± 0.7* |

### Industrial Applicability

The present invention is applicable for improving uric acid level, which is a case of trouble for middle aged people. The present invention can thus contribute to health.

## Claims

1. A composition comprising D-psicose as an active ingredient for use in lowering a uric acid level in a diabetes patient or a diabetes borderline person with fasting glucose level of 110 mg/dl or higher, wherein the composition being continuously administered in a dose of 5 g or more of D-psicose per intake in a daily dose of 15 g/day D-psicose for 2 weeks or longer than 2 weeks.

2. The composition for use according to claim 1, wherein the composition is a beverage, a food, a feed, a medicinal product, a quasi drug, an oral composition, or a beverage or food additive.

## Patentansprüche

1. Zusammensetzung, die D-Psicose als einen Wirkstoff enthält, für die Verwendung bei der Verringerung eines Harnsäurespiegels in einem Diabetespatienten oder einer Diabetes-Borderline-Person mit einem Nüchtern-Glukosespiegel von 110 mg/dl oder höher, wobei die Zusammensetzung kontinuierlich in einer Dosis von 5 g oder mehr D-Psikose pro Einnahme in einer täglichen Dosis von 15 g/Tag D-Psicose für 2 Wochen oder länger als 2 Wochen verabreicht wird.

2. Zusammensetzung für die Verwendung nach Anspruch 1, wobei die Zusammensetzung ein Getränk, ein Lebensmittel, ein Medizinprodukt, ein Quasi-Arzneimittel, eine orale Zusammensetzung oder ein Getränke- oder Lebensmittelzusatzstoff ist.

## Revendications

1. Composition comprenant du D-psicose à titre de principe actif, pour une utilisation dans l'abaissement du niveau d'acide urique chez un patient diabétique ou une personne prédiabétique limite ayant un niveau de glucose à jeun de 110 mg/dl ou plus, laquelle composition étant administrée en continu à une dose de 5 g ou plus de D-psicose par prise pour une dose quotidienne de 15 g/jour de D-psicose pendant 2 semaines ou plus de 2 semaines.

2. Composition pour une utilisation selon la revendication 1, laquelle composition est une boisson, un aliment, un aliment pour animaux, un produit médical, un quasi médicament, une composition à usage oral, ou un additif pour boisson ou pour aliment.
